# EUROPEAN PATENT SPECIFICATION

(11) **EP 1 304 574 B1**
(45) Date of publication and mention of the grant of the patent: **14.02.2007**
(21) Application number: 02425624.0
(22) Date of filing: 14.10.2002
(51) Int. Cl.: G01N 33/569

(54) **Elisa confirmation test for early primary cytomegalovirus infection**
Elisa Bestätigungstest für frühe Primarinfektion durch Zytomegaloviren
Test de confirmation Elisa pour l'infection primaire causée par le cytomegalovirus

(30) Priority: 18.10.2001 IT MI20012160
(43) Date of publication of application: 23.04.2003
(73) Proprietor: Marchisio, Edoardo, 20052 Monza (Milano) (IT)
(72) Inventor: Marchisio, Edoardo, 20052 Monza (Milano) (IT)

(56) References cited:
- WO-A-98/02746
- GREIJER ASTRID E ET AL: "Molecular fine-specificity analysis of antibody responses to human cytomegalovirus and design of novel synthetic-peptide-based serodiagnostic assays" JOURNAL OF CLINICAL MICROBIOLOGY, vol. 37, no. 1, January 1999 (1999-01), pages 179-188, XP002257029 ISSN: 0095-1137
- GRAHAM D A ET AL: "Detection of IgM responses to bovine respiratory syncytial virus by indirect ELISA following experimental infection and reinfection of calves: Abolition of false positive and false negative results by pre-treatment of sera with protein-G agarose" VETERINARY IMMUNOLOGY AND IMMUNOPATHOLOGY, vol. 71, no. 1, 1 October 1999 (1999-10-01), pages 41-51, XP002257030 ISSN: 0165-2427
- DATABASE MEDLINE [Online] US NATIONAL LIBRARY OF MEDICINE (NLM), BETHESDA, MD, US; December 1975 (1975-12) GISPEN R ET AL: "Immunofluorescence test for IgM rubella antibodies in whole serum after absorption with anti-gammaFc." Database accession no. NLM773577 XP002258654 & CLINICAL AND EXPERIMENTAL IMMUNOLOGY. ENGLAND DEC 1975, vol. 22, no. 3, December 1975 (1975-12), pages 431-437, ISSN: 0009-9104

### SEQUENCE LISTING

<110> Dia.Pro Diagnostic Bioprobes srl
   Edoardo, Marchisio
<120> Enzyme Linked Immuno Assay (ELISA) for the antigenic characterization of the early primary infection due to cytomegalovirus or CMV confirmation Test
<130> 02425624.0-1223/1304574
<140> 02425624.0-1223/1304574
   <141> 2003-05-26
<160> 8
<170> PatentIn version 3.1
<210> 1
   <211> 38
   <212> PRT
   <213> Cytomegalovirus
<300>
   <301> G. Jahn et al.
   <302> Prokariotic expression of immunogenic polypeptides of the large phosphoprotein (pp150) of human cytomegalovirus
   <303> Journal of virology
   <304> 69
   <305> 6
   <306> 1195-1204
   <307> 1988-06-01
   <308> PMID 2455019
   <309> 1988-06-01
<400> 1
<210> 2
   <211> 40
   <212> PRT
   <213> cytomegalovirus
<300>
   <301> Jahn G. ; scholl B.C.
   <302> Prokariotic expression of immunogenic polypeptides of the large phosphoprotein (pp150) of human cytomegalovirus
   <303> J. Gen. virology
   <304> 69
   <305> 6
   <306> 1195-1204
   <307> 1988-06-01
   <308> PMID 2455019
   <309> 1988-06-01
<400> 2
   Leu Thr Pro Thr Pro Val Asn Pro Ser Thr Ala Pro Ala Pro Ala Pro
   1               5                   10                  15
   Thr Pro Thr Phe Ala Gly Thr Gln Thr Pro Val Asn Gly Asn Ser Pro
               20                  25                  30
   Trp Ala Pro Thr Ala Pro Leu Pro
           35                  40
<210> 3
   <211> 13
   <212> PRT
   <213> Cytomegalovirus
<300>
   <301> Kari B. ; Radeke B.
   <302> The human cytomegalovirus UL100 gene encodes the gC-II glycoprotein recognized by group 2 monoclonal antibodies
   <303> J. Gen. Virology
   <304> 75
   <305> 11
   <306> 3016-3018
   <307> 1994-11-01
   <308> PMID 7964617
   <309> 1994-11-01
<400> 3
   Met Ala Pro Ser His Asp Lys Val Asn Thr Arg Thr Trp
   1               5                   10
<210> 4
   <211> 20
   <212> PRT
   <213> cytomegalovirus
<300>
   <301> Kari B. ; Radeke B.
   <302> The human cytomegalovirus UL100 gene encodes the gc-1II glycoprotein recognized by group 2 monoclonal antibodies
   <303> J. Gen. virology
   <304> 75
   <305> 11
   <306> 3016-3018
   <307> 1996-11-01
   <308> PMID 7964617
   <309> 1994-11-01
<400> 4
   Ala Ser Gly Glu Glu Val Ala Val Leu Ser His His Asp Ser Leu Glu
   1 5 10 15
   Ser Arg Arg Leu
   20
<210> 5
   <211> 35
   <212> PRT
   <213> Cytomegalovirus
<300>
   <301> Leach F.s. ; Mocarski E.S.
   <302> Regulation of the cytomegalovirus late gene expression differential use of three start sites in the trascriptional activation of ICP36 gene expression <303> J. virology
   <304> 63
   <305> 4
   <306> 1783-1791
   <307> 1989-04-01
   <308> PMID 2538657
   <309> 1989-04-01
<400> 5
   Leu Asp Arg Asn Ser Gly Asn Tyr Phe Asn Asp Ala Lys Glu Glu Ser
   1 5 10 15
   Asp Ser Glu Asp Ser Val Thr Phe Glu Phe Val Pro Asn Thr Lys Lys
   20 25 30
   Gln Lys Cys
   35
<210> 6
   <211> 25
   <212> PRT
   <213> Cytomegalovirus
<300>
   <301> Meyer H. ; Mach M.
   <302> Identification and proKariotic expression of the gene encoding for the highly immunogenic 28- Kd structural phosphoprotein (pp28) of human Cytomegalovirus
   <303> J. Virology
   <304> 62
   <305> 7
   <306> 2243-2250
   <307> 1988-07-01
   <308> PMID 2836608
   <309> 1988-07-01
<400> 6
   Glu Phe Gly Thr Thr Pro Gly Glu Pro Leu Lys Asp Ala Leu Gly Arg
   1               5                   10                  15
   Gln Val Ser Leu Arg Ser Tyr Asp Asn
               20                  25
<210> 7
   <211> 25
   <212> PRT
   <213> CytomegaTovirus
<300>
   <301> David M. G. ; Huang E.S.
   <302> Mapping and expression of human cytomegalovirus major viral protein <303> J. virology
   <304> 52
   <305> 1
   <306> 129-135
   <307> 1984-10-01
   <308> PMID 6090690
   <309> 1984-10-01
<400> 7
   Pro Ser Leu Ser Glu Lys Lys Thr Ala Ser Pro Thr Cys Val Lys His
   1               5                   10                  15
   His Leu Ser Gly Gly Ala Val Arg Arg
               20                  25
<210> 8
   <211> 25
   <212> PRT
   <213> Cytomegalovirus
<300>
   <301> Davis M.G. ; Huang M.S.
   <302> Mapping and expression of human cytomegalovirus major viral protein <303> J. virology
   <304> 52
   <305> 1
   <306> 129-135
   <307> 1984-10-01
   <308> PMID 6090690
   <309> 1984-10-01
<400> 8
   Gly Arg Ser Arg Pro Thr Thr Phe His Leu Thr Arg Lys Lys Lys Ala
   1 5 10 15
   Pro Leu Gly Gly Asp Leu Ser Pro Ser
   20 25

## Description

This invention relates to a process for the determination of IgM class antibodies, produced by the immune system of a patient during Cytomegalovirus primary infection or its reactivation, in human biological fluids by means of an Enzyme Linked Immunosorbent Assay or ELISA through the use of a series of immunodominant and specific peptides. The peptides used are those of claim 1.

Human Cytomegalovirus belongs to the Herpesviridae familiy and its infection spread from human to human through contaminated blood and body fluids such as urine, saliva, semen, breast milk and cervical secretions.

The class IgM antibodies detection is currently a powerful diagnostic tool for monitoring pregnant women and risky patients (mainly transplanted and AIDS patients), exposed to CMV infection.

A CMV acute primary infection represents an important cause of risk of fetus abnormalities and death for immune compromised and immune suppressed patients.

Even if the current immunodiagnostic assays have increased the chances of a faster and more accurate diagnosis of CMV infection, the medical literature points out some important problems regarding the determination of IgM antibodies produced by the infected organism against CMV in the first steps of the primary infection or in the reactivation of a chronic infection.

On account of the high variability of antigens expressed during the CMV life cycle and to the complexity of the immune response developed by the patient, discrepancies in results coming from different laboratory tests is often observed mainly due to the high variance in the quality of the test used or the analytical methodology applied.

Discrepancy may lead to wrong and harmful evaluations of the clinical status of the patient under monitoring and to the risk of serious mistakes in selecting the best pharmacological therapy.

The immunoenzymatic has been developed aimed at:
1. Confirming the diagnosis of CMV primary infection when in presence of the above mentioned discrepancies in the diagnostic test results or in case of border line results, difficult to be interpretatated.
2. Supplying more reliable results in case of "difficult" samples coming from risk patients.
3. Providing a wider view on the clinical situation of the patient and about the infection progressive developing.

### Principle of the assay

The CMV confirmation Kit is based on an ELISA microplate made of 12 strip modules. Each strip-module is composed of 8 reaction microwells and is dedicated to test one sample (mainly serum or plasma).

Each microwell, the strip-module is composed of, is coated with one or more synthetic antigens bearing CMV specific sequences. These are derived from conserved and immunodominant epitopes coming from CMV antigens showing an important role in the diagnosis of the disease and well described by the scientific literature.

The microwells of the strip-module are coated with the specific antigens according to the following scheme :

| **Position** | **Protein** | **Function** |
|---|---|---|
| A | Casein | Negative Control |
| B | IgG Anti hIgM | Positive Control |
| C | UL 32 or Phosphoprotein 150 region 1 | IgM test |
| D | UL 32 or Phosphoprotein 150 region 2 | IgM test |
| E | UL 100 or Glicoprotein M | IgM test |
| F | UL 44 or Phosphoprotein 52 | IgM test |
| G | UL 99 or Phosphoprotein 28 | IgM test |
| H | Phosphoprotein 67 | IgM test |

The scheme, the test is based on, derives from the well-known RIBA and Western-Blot assay where native antigens, bound on a nylon or nitrocellulose support, give a deep view about the immune reactivity status of the patients.

The sample (mainly serum or plasma) is treated with an immunsorbent (Protein G Agarose) able to bind and remove possible interferences due to IgG class antibodies or to the Rheumatoid Factor (RF).

The sample treated with the immunosorbent reagent is dispensed in each microwell of the Strip-Module.

During the first incubation of the assay, IgM antibodies, if present in the sample, are specifically captured by synthetic antigens coated on the microwell.

After washing out not specific antibodies, anti CMV IgM antibodies are detected by the addition of a polyclonal antibody, IgM specific, affinity purified and conjugated to horseradish peroxidase (HRP).

After washing to remove the unbound tracer, each microwell is dispensed with a solution composed by a substrate and a chromogenic molecule which due to his reaction with the enzyme (HRP) develops an optical signal, directly proportional to the quantity of IgM specifically bound to CMV antigens.

The optical signal is read by a spectrophotometer at a specific wave length (450 - 650 nm) and results elaborated by a dedicated software.

### Main features of the invention

The invention is related to an immunoenzymatic (ELISA) assay for the analysis of the immune status produced upon a CMV primary infection or reactivation by determining the IgM class antibodies specific to the CMV immunodominant and conserved antigens of claim 1, absorbed on a micro well titer plate.

The present invention can ease the physicians to define, with a high degree of confidence, an active CMV infection or a reactivation, through the characterization of the IgM antibody response.

This invention can moreover help the physician to make a diagnosis of CMV infection and to define a therapy against it in an easier, more reliable and faster way.

### 1. Characterization and choice of the polypeptide sequences

The choice of the synthetic peptides to activate the wells of the strip-module has been done on the basis of these technical reasons :
1. Synthetic peptides represent an antigenic reagent of high specificity;
2. Synthetic peptides are produced with reliable technical procedures and with an high degree of lot-to-lot homogeneity;
3. Synthetic peptide, when correctly selected, can bear highly immunogenic sequences of CMV antigens, coming from important epitopes against which the specific immune response is directed;
4. Synthetic peptides let overcome problems due to the use of antigenic preparations of CMV obtained from in vitro tissue culture systems, usually composed by host cells of human origin, that could be contaminated by human proteins
5. Synthetic peptides let overcome problems of crossreactions with other viruses of the family of Herpesviridae (HSV, EBV) by selecting CMV specific sequences.

Each synthetic peptide, present in this assay, represents unequivocally a CMV specific sequence derived from CMV antigens expressed during the early steps of virus replication and known to be stimulating the production of IgM antibodies.

A deep study has been conducted in the scientific literature to identify the presence of protein with amino acidic sequences well identified and characterized to be clinically and immunologically relevant in the CMV infection.

We have identified and selected a list of scientific publications from which we have extracted information about CMV proteins structure and immunogenicity useful for the development of the assay, as follows :
**1. K. S. Kim, V.J. Sapienza, R.I. Carp and H.M.** Moon
   Analysis and structural polypeptydes of purified human cytomegalovirus.
   Journal of Virology, Dec. 1976, p. 604-611
**2. N.E. Cremer, C.K. Cossen, G.R. Shell and L.** Pereira
   Antibody response to cytomegalovirus polypeptides captured by monoclonal antibodies on solid phase in enzyme immunoassay.
   Journal of Clinical Microbiology, Apr. 1985, p. 517-521
**3. M.P. Landini and M. La Placa**
   Humoral immuno response to human cytomegalovirus proteins : a brief review.
   Comp.Immun. Microbiol. Infect. Dis. Vol.14, n°2, p97-105, 1991
***4.******Middeldorp Jaap Michiel ;** AKZO NOBEL NV (NL)* ; *Crommert Johannes Mar) 22 January 1998 (1998-01-22) WO 98 02746*
***5.******Greijer Astrid E. et Al** : « Molecular fine-specificity analysis of antibody responses to human cytomegalovirus and design of novel syntheic-peptide-based serodiagnostic assays*"
   *Journal of clinical microbiology, vol.* 37, *no. 1, January* 1999 *(1999-01), pages 179-188*, *XP002257029 ISSN: 0095-1137*

In these publications we identified the CMV specific proteins of major interest under a diagnostic point of view, suitable for the determination of the IgM class antibodies during a primary CMV infection or reactivation, as follows :
1. Phosphoprotein pp150 o UL 32
2. Glicoprotein M o UL 100
3. Phosphoproteine pp52 o UL 44
4. Phosphoprotein pp28 o UL 99
5. Phosphoprotein pp67

The bibliographic documentation specific on immunodominant antigens was obtained from the following scientific publications:
**1**. **pp150**
   G. Jahn et al. Journal of Virology 1988, 62:
   p. 2243- 2250
**2. gp M**
   B. Kari et al. Journal of General Virology 1994, 75: p.3081-3086
**3. pp 52**
   Leach F.S. ,Mocarsky E.S. J.Virol. 63: 1783-1791 (1989)
**4. pp 28**
   H.Meyer et al. Journal of Virology 1988, 62: p. 2243-2250
**5. pp 67**
   M. G. Davis et al. Journal of Virology 1985, 56: p.7-11

Each antigen, identified as a potential reactive agent for the solid phase represented by wells of the microplate, has been studied for the immunological characteristics of its aminoacidic sequence, analyzing it with informatics tools as the programs "Geneworks" and "Peptide Companion", or on the basis of the data found in the publications mentioned above.

Identified the most interesting sequences, we have proceeded to synthesize them by a fully automated peptide synthesizer, by means of "F-Moc" or " T-Boc" solid phase chemistry and to peptides purification by "reverse phase" HPLC procedure.

Each peptide has been analyzed and characterized by a panel of well-defined CMV IgM positive samples, pre-tested in a Elisa systems for CMV IgM detection (Abbott and Dia.Pro code CMVM2).

Results have lead to the identification of the sequences used in our immunoenzimatic system, enumerated as follows in succession:

### Antigen pp 150

1) Sequence pp150-1 AA 1011-1048
   KSGTGPQPGSAGMGGAKTPSDAVQNILQKIEKIKNTEE -cooh
2) Sequence pp150-2 AA 594-633
   LTPTPVNPSTAPAPAPTPTFAGTQTPVNGNSPWAPTAPLP -cooh

### Antigen gp M

1) Sequence gpM-1 AA 1-13
   MAPSHDKVNTRTW-cooh
2) Sequence gpM-2 AA 292-311
   ASGEEVAVLSHHDSLESRRL- cooh

### Antigen CMV pp52

1) Sequence pp 52 AA 397-432
   LDRNSGNYFNDAKEESDSEDSVTFEFVPNTKKQKC-cooh

### Antigen CMV pp 28

1) Sequence pp28 AA 7-31
   EFGTTPGEPLKDALGRQVSLRSYDN-cooh

### Antigen CMV pp67

1) Sequence pp 67-1 AA 82-106
   PSLSEKKTASPTCVKHHLSGGAVRR-cooh
2) Sequence pp 67-2 AA 38-62
   GRSRPTTFHLTRKKKAPLGGDLSPS-cooh

The amino acids are identified in the list by an international single letter code according to the following reading key:

A = Alanine, R = Arginine , N= Asparagine , D = Aspartic acid , C = Cysteine , Q = Glutamine , E = Glutamic acid , G = Glycine , H = Histidine , I = Isoleucine , L= Leucine, K = lysine , M = methionine, F = Phenylalanine, P = Proline, S = Serine, T = Threonine , W = Tryptophan, Y = Tyrosine , V = Valine

### 2. Micro plate activation procedure

A 96 microwells polystyrene titer plate, composed by 12 strips-modules each made by 8 wells, is used for the assay.

Each strip is considered as a test module useful to carry out the analysis of one sample. Each well in a strip-module is coated with one or more synthetic antigens in a concentration determined through a series of previous analysis.

Antigens coated according to the following scheme, considering to start from the first well on the upper side down to the lower side of the strip module:

| | | | |
|---|---|---|---|
| A. | casein used as negative control in the system | | |
| B. | Policlonal antibody to human IgM used as positive control in the system | | |
| C. | Phosphoproteine 150 | 1 ^ sequence | IgM test |
| D. | Phosphoproteine 150 | 2^ sequence | IgM test |
| E. | Glycoprotein M | 1^ & 2^ sequences | IgM test |
| F. | Phosphoproteine 52 | | IgM test |
| G. | Phosphoproteine 28 | | IgM test |
| H. | Phosphoproteine 67 | 1^ & 2^ sequences | IgM test |

The synthetic antigens are diluted to the chosen concentration in a 10mM

Na-carbonate buffer, adjusting the pH to 9.4 +/-0.2 using Na-bicarbonate and then antigens are dispensed into the proper well.

The microplate, after an overnight incubation at +37 °C are washed with a 0.1% solution of Tween 20 in water and then incubated for 4 hour at room temperature (20-25° C) with 200 ul of an overcoating solution composed of 0.2 M Tris base, 2% idrolized Casein and HCI to pH of 7.6 +/-0.2.

After the last incubation, the solution present in the microwell is aspirated , the solid phase is dried and finally sealed into an aluminum bag with a desiccant material.

### 3. Method for the elimination of the interference caused by IgG antibodies and Rheumatoid Factor (RF)

In order to get reliable results, the role of a procedure for the elimination of the false reactivity from the system by using an absorbing reagent (Protein G Agarose) is essential *and it is well described in Graham D. A. et Al.: "Detection of IgM responses to bovine respiratory syncytial virus by indirect ELISA following the experimental infection and reinfection of calves : abolition of false positive and false negative results by pre-treatment of sera with protein-G Agarose" VETERINARY IMMUNOLOGY AND IMMUNOPATHOLOGY, vol 71, no. 1,1 October 1999 (1999-10-01), pages 41-51, XP002257030 ISSN: 0165-2422 This step is used in the assay to capture the class IgG immunoglobulin present in serum and plasma, avoiding the possibility of their interference in the test*

The method of sample treatment has been optimized by a filtrating unit, proceeding as described in the following scheme:
1. Serum or plasma is diluted in a protein buffered solution together with the adsorbent reagent for a fixed time, defined previously, under stirring;
2. Separation of Protein G Agarose from the suspension is then achieved by pushing it through a "Filtering Piston" able to release only the liquid part of the suspension, containing the IgM to be tested ;
3. IgM to be examined for anti CMV reactivity are then recovered in the upper reservoir of the "Filtering Piston", sipped out with a pipette and then transferred into a test vial.

The protein G Agarose used in the system has the following characteristics:
1. It is conjugated with spherical microparticles of agarose with a diameter comprised from 45 to 165 um ( Micrometer);
2. It is activated with approximately 2 milligrams (mg) of protein G per ml (milliliter) of the gel;
3. It has a binding capacity of about 20 mg (milligrams) of class IgG antibodies per ml of gel.

The procedure for the elimination of the interfering substances from the samples is then achieved by the following steps:
1. 10 ul of the serum or plasma under evaluation are diluted in reaction vial with 1 ml of 0.1 M Tris, 0.3 M Nacl, 1% Casein, 0.1% Kathon GC and HCI pH 7.4 +/-0.2, containing 200 ul of Protein G Agarose.
2. The suspension, containing the sample diluted 1:100, is mixed on vortex for one minute and then the gel is left to sediment for one minute. This step is carried out at room temperature.
3. A " Filtering Piston" with a porous filter of about 40 micron is pushed manually into the reaction vial separating the absorbent reagent from the IgM antibodies to test.
4. The filtrated solution is aspirated by a plastic pipette from the reservoir of the "Filtering Piston" and transferred to a test vial for the determination.

### 4. Neutralizing reagent

Aimed at obtaining the highest rate in the elimination of interferences by IgG Antibodies or Rheumatoid Factor (RF), a second procedure was further introduced in the assay, based on the use of a reagent specific for their neutralization *as described in GISPEN R et Al.: "Immunofluorescence test for IgM rubella antibodies in whole serum after absorption with (gamma)Fc' CLINICAL AND EXPERIMENTAL IMMUNOLOGY 1975, vol. 22, no. 3,1975, pages 431-437, XP002258654 1985 (1985-10-01), pages 7-11, XP002038724 ISSN: 0012 538X.* The neutralizing reagent is dispensed in the microwells of the strip-module before dispensing the sample of serum or plasma under evaluation.

The formulation of the neutralizing reagent is the following:

| | |
|---|---|
| diNa -Phosphate dodecaidhrate | 0.01 M |
| NaCl | 0.15 M |
| Antiserum anti hFc IgG | 30 % |
| o-Phosphoric acid to pH 7.4 +/-0.2 | |

The Neutralizing reaction is carried by adding 50 ul of Neutralizing Reagent into each well of a module, just before adding 50 ul of the sample previously treated with protein G Agarose.

The time sequence of the adding is strategic for the right execution of the test procedures as, if the neutralizing reagent is added after the sample, it could not inactivate any interfering IgG, eventually escaped from the immunoadsorption procedure .

### 5. Test procedure

After treating the serum or plasma with the Immunoadsorbent Reagent followed by the addition of the Neutralizing Reagent, 50 ul of the sample is added to each well of the strip-module.

The module is then incubated for 45 minutes in a ELISA thermostatic incubator at the controlled temperature of +37°C +/-1.

At the end of the incubation, the strip-module is washed for 5 times with 300 ul of washing buffer, previously diluted 1:20 with ELISA grade water, by means of an automatic washer.

The washing buffer, 20X concentrated, has the following composition:

| | |
|---|---|
| Na-Phosphate, bibasic | 10 mM |
| NaCl | 0.15 M |
| Tween 20 | 0.05% |
| Kathon GC | 0.1% |
| HCl to pH 7.4 +/-0.2 | |

In each well of the strip-module 100 ul of an enzymatic tracer composed of a polyclonal antibody specific to human IgM, purified by affinity chromatography and labeled with Horseradish peroxidase (HRP).

The enzyme conjugate, supplied 20X concentrated and titrated to provide the best performances, is diluted few minutes before in a buffer containing 0.1 M Tris-base, 2% Bovin Albumin, 0.1% Kathon GC, 2 mg/ml Gentamycine sulphate, and Citric Acid to pH 6.5 +/-0.1.

The strip-module is then incubated at 37°C for 45 minutes.

When the second incubation is over, the module is washed as previously described. Then 100 ul of a stabilized solution of a chromogen (tetramethylbenzidine or TMB) and a substrate (hydrogen peroxide or H₂O₂), supplied ready to use, are dispensed in all the wells of the strip-module.

The enzymatic tracer bound to the IgM, captured specifically on the microwells, by reacting with the chromogen/substrate solution develops a color whose intensity is proportional to the quantity of CMV specific IgM present in the sample.

The enzymatic reaction is stopped after an incubation of 15 minutes in the dark at room temperature by the addition of 100 ul of 0.3 M sulphuric acid in all the wells of the strip-module .

The absorbance of the colored solution generated by the enzymatic reaction is read with spectrophotometer at 450 nm (reading filter) and possibly at 640-650 nm (blank filter) in order to subtract any false absorbance due to finger prints and dust, eventually present on the plastic.

### 6. Interpretation of results

The optical densities coming from the photometric readings, subtracted of the 654-650nm values, are considered valid if they match the following parameters :
1. OD 450nm value of the "A" well less than 0,300.
2. OD 450nm value of the "B " well higher than 1,500.

If the validity of the test is confirmed, results from the other wells of the strip-module can be interpreted according to the following scheme:
1. Negative result: if wells "C,D,E,F,G,H" show OD values lower than the OD450nm of "A" + 0,250
2. Equivocal result: If the OD450nm of well "A" is higher than 0,300.
3. Positive result: if at least one well out of "C,D,E,F,G,H" shows an OD value higher than the OD450nm of "A" + 0,250

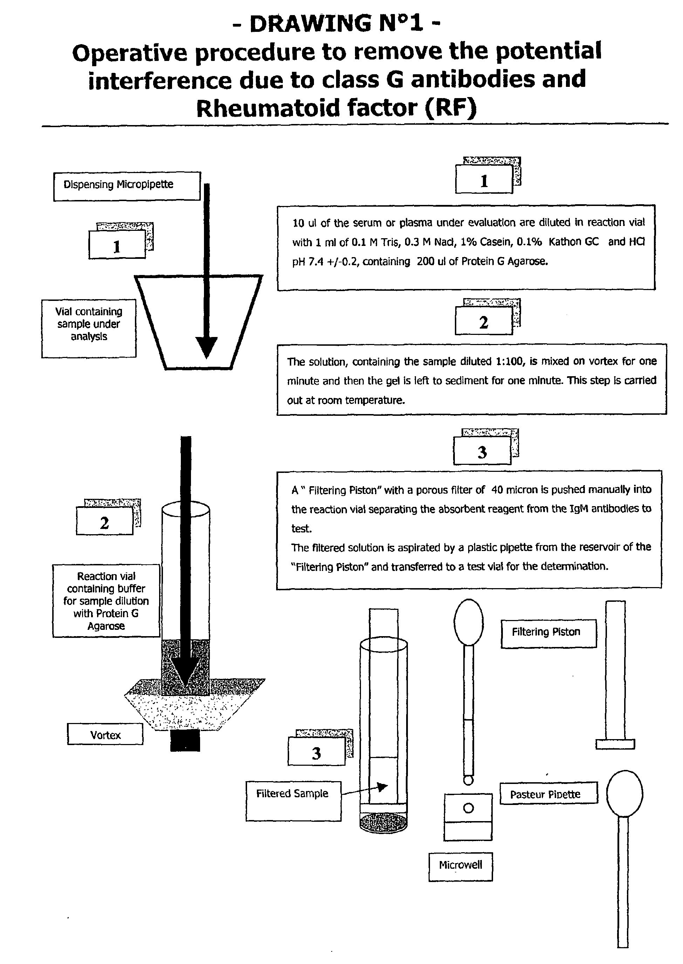

## Claims

1. A process for the determination of IgM class antibodies, produced by the immune system of a patient during Cytomegalovirus primary infection or its reactivation, in human biological fluids by means of an Enzyme Linked ImmunoSorbent Assay or ELISA, which makes use of CMV specific synthetic peptides of the following sequences:
**Phosphoprotein 150 (pp150)**
1^sequence aa 1011-1048
KSGTGPQPGSAGMGGAKTPSDAVQNILQKIEKIKNTEE-cooh
2^sequence aa 594-633
LTPTPVNPSTAPAPAPTPTFAGTQTPVNGNSPWAPTAPLP-cooh,
**Glicoprotein M (gpM)**
1^sequence aa 1-14
MAPSHDKVNTRTW-cooh
2^sequence aa 292-311
ASGEEVAVLSHHDSLESRRL- cooh,
Phosphoprotein 52 (pp52)
sequence aa 397-432
LDRNSGNYFNDAKEESDSEDSVTFEFVPNTKKQKC-cooh,
Phosphoprotein 28 (pp28)
sequence aa 7-31
EFGTTPGEPLKDALGRQVSLRSYDN-cooh,
and
**Phosphoprotein 67 (pp67)**
1^sequence aa 82-106
PSLSEKKTASPTCVKHHLSGGAVRR-cooh
2^sequence aa 38-62
GRSRPTTFHLTRKKKAPLGGDLSPS-cooh.

2. A process according to Claim 1 which makes use of a test-module made of a polystyrene ELISA microplate strip, as solid phase. The strip is composed of 8 flat bottom wells and the module is sufficient for the analysis of one sample ;

3. A process according to Claim 2 wherein twelve test-modules are supplied firmly held into a ELISA microplate ;

4. A process according to Claim 1 which is based on a test-module whose wells are singularly activated with different synthetic peptides, able to provide negative and positive internal assay controls and CMV specific IgM determinations ;

5. A process according to Claim 1 wherein micro-wells of a test-module are activated according to the following scheme:
| **Position** | **Protein** | **Function** |
|---|---|---|
| A | Casein | Negative Control |
| B | IgG Anti hIgM | Positive Control |
| C | UL 32 or Phosphoprotein 150 region 1 | IgM test |
| D | UL 32 or Phosphoprotein 150 region 2 | IgM test |
| E | UL 100 or Glicoprotein M | IgM test |
| F | UL 44 or Phosphoprotein 52 | IgM test |
| G | UL 99 or Phosphoprotein 28 | IgM test |
| H | Phosphoprotein 67 | IgM test |

6. A process according to Claim 1 which includes a pre-treatment of the biological specimen during which the interfering class IgG antibodies which can give false reactions mostly in combination with the rheumatoid factor are captured by means of Protein G Agarose and removed from the specimen through the following procedure and materials :
a. 10 *µ*l of biological fluid under analysis which is mainly serum or plasma, is added to a reaction vial containing 1 ml (milliliter) of 0.1 M Tris base, 0.3 M NaCl, 1% Casein, 0.1% Kathon GC and HCI to pH 7.4, in which 200 *µ*l of 0.5 mg/ml Protein G Agarose have been previously added. The suspension, in which the sample has been diluted to the final dilution of 1:100, is mixed on vortex for at least one minute. The suspension is left to sediment for one minute at 20-25°C.
b. A filtering piston with a filter with 40 micrometers sized pores is pushed with a finger tip pressure into the reaction vial separating the adsorbent reagent Protein G Agarose to the bottom of the vial from the aqueous solution containing the IgM to test, collected in the reservoir of the filtering piston.
c. The filtered solution collected in the reservoir is aspirated by a plastic pipette and transferred to a new vial waiting the determination.

7. A process according to Claim 1 which uses a neutralizing reagent which removes any residual presence of interfering class IgG antibodies, eventually remained in the solution despite the treatment with Protein G Agarose. This step is carried out by adding 50 *µ*l of the neutralizing reagent directly in the microwells of the reaction strip-module just before the addiction of the sample, diluted and pre-treated as described to avoid interferences due to the class IgG antibodies before they could react with the peptides on the solid phase;

8. A process according to Claim 7 wherein the neutralizing reagent has the following formula :
| | |
|---|---|
| diNa-Phosphate dodecaidhrate | 0.01M |
| NaCl | 0.15M |
| Anti hIgG-Fc specific antiserum | 30% |
| HCl to pH 7.4 +/-0.1 | |

## Patentansprüche

1. Es handelt sich um ein Verfahren, um die IgM Klasse Antikörper zu bestimmen, die durch das Immunsystem eines Patienten während Cytomegalovirus- primäre Infektion oder ihre Reaktivierung in menschlichen biologischen Flüssigkeiten mittels einer Enzym verbundenen ImmunoSorbent Analyse ELISA erzeugt wird. Es verwertet CMV bestimmte synthetische Peptiden laut folgender Abfolge (Sequences):
Phosphoprotein 150 (pp150)
1. Abfolge (Sequences): aa 1011-1048
KSGTGPQPGSAGMGGAKTPSDAVQNILQKIEKIKNTEE-cooh
2. Abfolge (Sequences): aa 594-633
LTPTPVNPSTAPAPAPTPTFAGTQTPVNGNSPWAPTAPLP-cooh,
Glycoprotein M (gpM)
1. Abfolge (Sequences): aa 1-14
MAPSHDKVNTRTW-cooh
2. Abfolge (Sequences): aa 292-311
ASGEEVAVLSHHDSLESRRL- cooh
Phosphoprotein 52 (pp52)
Abfolge (Sequences): aa 397-432
LDRNSGNYFNDAKEESDSEDSVTFEFVPNTKKQKC-cooh,
Phosphoprotein 28 (pp28)
Abfolge (Sequences): aa 7-31
EFGTFPGEPLKDALGRQVSLRSYDN-cooh,
Und
Phosphoprotein 67 (pp67)
1. Abfolge (Sequences): aa 82-106
PSLSEKKTASPTCVKHHLSGGAVRR-cooh
2. Abfolge (Sequences): aa 38-62
GRSRPTTFHLTRKKKAPLGGDLSPS-cooh.

2. Es handelt sich um Patent 1, das mit einem Polyster ELISA mikroplate- Streifen zusammengesetzten Probe-Modul benutzt; feste Phase. Der Streifen besteht aus 8 flachen Grund-Wells und das Modul reicht, um ein Muster einer Analyse zu unterziehen;

3. Ein Verfahren gemäss Patent 2, in dem zwölf Probe-Module versorgt werden, die ganz fest in einem ELISA Mikroplate enthalten sind:

4. Ein Verfahren gemäss Patent 1, das auf einem Probe-Module beruht, dessen Wells separat mit verschiedenen synthetischen Peptiden aktiviert werden. Solche Peptiden können negative und positive innere Bewertungskontrollen, sowie CMV bestimmte IgM Bestimmungen versehen;

5. Ein Verfahren gemäss Patent 1, in dem die Mikrowells eines Probe-Moduls aufgrund der folgenden Tabelle aktiviert sind:
| **Stellung** | **Protein** | **Funktion** |
|---|---|---|
| A | Casein | Negative Control |
| B | IgG Anti hIgM | Positive Control |
| C | UL 32 or Phosphoprotein 150 region 1 | IgM test |
| D | UL 32 or Phosphoprotein 150 region 2 | IgM test |
| E | UL 100 or Glicoprotein M | IgM test |
| F | UL 44 or Phosphoprotein 52 | IgM test |
| G | UL 99 or Phosphoprotein 28 | IgM test |
| H | Phosphoprotein 67 | IgM test |

6. Ein Verfahren gemäss Patent 1 mit einer Vorbehandlung der biologischen Mustern, in der die Klasse IgG interferierten Antikörper falsche Reaktionen verursachen können, meistens in Verbindung mit rheumatischen Faktoren. Die Antikörper werden durch das Protein G-Agarose blockiert und von den Mustern entfernt laut des folgenden Verfahrens und der folgenden Materialen:
a. 10 µl biologischer Flüssigkeit wird analysiert; das besteht hauptsächlich aus Serum und Plasma und wird einer Reaktion hinzugefügt mit 1 ml von 0.1 M Tris Base , 0,3 M NaCI, 1% Casein, 0,1% Kathon GC und HCI zu pH 7.4, in dem 200µ von 0,5 mg/ml Protein G-Agarose sind vorher hinzugefügt worden. Die Suspension, in der das Muster bis zur endlichen Verdünnung von 1:100 gestreckt worden ist, wird auf Vortex für mindestens eine Minute vermischt. Die Suspension soll für eine Minute zu 20-25° C abgelagert werden.
b. Ein Filterventil mit 40 Mikrometern Poren wird beim Drücken der Fingerspitzen in die Reaktion Vial geschoben. Dieses Verfahren scheidet das absorbierende Reaktionsmittel Protein G-Agarose bis auf den Grund des Vials aus der Wasserlösung aus, mit dem IgM zum Testen.
Diese Lösung wird im Behälter des Filterventils bewahrt.
c. Die Filterlösung im Behälter wird durch einer Kunstpipette abgesaugt und dann in einen neuen Vial eingeflossen, damit sie später bestimmt werden kann.

7. Ein Verfahren gemäss Patent 1 mit einem neutralisierenden Reaktionsmittel, das jedes Zeichen von Klasse IgG interferierten Antikörpern in der Lösung trotz der Behandlung mit dem Protein G Agarose entfernen kann. Diese Phase wird ausgeführt, indem 50 µl des neutralisierenden Reaktionsmittels unmittelbar in die Mikrowells der Streifen-Modul Reaktion hinzugefügt werden kurz bevor das Muster hinzugefügt wird. Das soll verdünnt und vorbehandelt werden, so dass keine Einmischung infolge der Klasse IgG Antikörper vorkommt, bevor sie mit Peptiden auf der festen Phase reagieren können:

8. Ein Verfahren gemäss Patent 7, in dem das neutralisierende Reaktionsmittel weist die folgende Formel auf:
| | |
|---|---|
| diNa- Phosphate dodecaidhrate | 0.01M |
| NaCI | 0.15M |
| Anti hIgG-Fc spezifische Anti-Serum | 30% |
| HCI zu Ph 7.4 +/-0.1 | |

## Revendications

1. Une méthode pour la détermination d'anticorps de la classe IgM, produit par le système immunitaire d'un patient pendant l'infection primaire de Cytomegalovirus ou bien sa réactivation, dans des fluides biologiques humains au moyen d'ELISA (Enzyme Linked ImmunoSorbent Assay) qui emploie des peptides synthétiques spécifiques CMV selon les suivantes séquences :
Phosphoprotéine 150 (pp150)
1^{ère} séquence aa 1011-1048
KSGTGPQPGSAGMGGAKTPSDAVQNILQKIEKIKNTEE-cooh
2^{ème} séquence aa 594-633
LTPTPVNPSTAPAPAPTPTFAGTQTPVNGNSPWAPTAPLP-cooh,
Glicoprotéine M (gpM)
1^{ère} séquence aa 1-14
MAPSHDKVNTRTW-cooh
2^{ème} séquence aa 292-311
ASGEEVAVLSHHDSLESRRL- cooh,
Phosphoprotéine 52 (pp52)
Séquence aa 397-432
LDRNSGNYFNDAKEESDSEDSVTFEFVPNTKKQKC-cooh,
Phosphoprotéine 28 (pp28)
Séquence aa 7-31
EFGTTPGEPLKDALGRQVSLRSYDN-cooh,
et
Phosphoprotéine 67 (pp67)
1^{ère} séquence aa 82-106
PSLSEKKTASPTCVKHHLSGGAVRR-cooh
2^{ème} séquence aa 38-62
GRSRPTTFHLTRKKKAPLGGDLSPS-cooh.

2. Une méthode conforme à la déclaration 1 qui emploie pour la phase solide un module pour essais fait d'une bande de microplaques en polystyrène ELISA. La bande est composée de 8 puits à fond plat et le module est suffisant pour l'analyse d'un échantillon ;

3. Une méthode conforme à la déclaration 2 qui prévoit 12 modules pour essais solidement retenus dans une microplaque ELISA ;

4. Une méthode conforme à la déclaration 3 qui se base sur un module pour essais les puits duquel sont activés singulièrement par des différents peptides synthétiques, capables d'assurer des contrôles positifs et négatifs pour les essais internes et aussi déterminations IgM spécifiques pour CMV ;

5. Une méthode conforme à la déclaration 1 qui prévoit que les micro puits d'un module pour essais soient activés selon le schéma suivant
| Position | Protéine | Fonction |
|---|---|---|
| A | Casein | Negative Control |
| B | IgG Anti hIgM | Positive Control |
| C | UL 32 or Phosphoprotein 150 region 1 | IgM test |
| D | UL 32 or Phosphoprotein 150 region 2 | IgM test |
| E | UL 100 or Glicoprotein M | IgM test |
| F | UL 44 or Phosphoprotein 52 | IgM test |
| G | UL 99 or Phosphoprotein 28 | IgM test |
| H | Phosphoprotein 67 | IgM test |

6. Une méthode conforme à la déclaration 1 qui inclut un prétraitement de l'échantillon biologique pendant lequel les anticorps de la classe IgG interférents qui peuvent donner des réactions fausses généralement en combinaison avec le facteur rhumatoïde sont capturés grâce à la protéine G Agarose et éliminés de l'échantillon au moyen des suivants procédures et matériels :
a. 10 µl de fluide biologique sous analyse, qui est principalement sérum ou plasma, est ajouté à une ampoule de réaction contenant 1 ml (millimètre) de 0.1 M Tris Base, 0.3 M NaCl, 1% caséine, 0.1 Kathon GC e HCl à pH 7.4, dans lequel on a déjà ajouté 200 µl de Protéine G Agarose 0.5 mg/ml. La suspension dans laquelle l'échantillon a été dilué jusqu'à la dilution finale de 1 :100 est mélangé sur vortex pendant une minute au moins. La suspension est laissée sédimenter pour une minute à 20-25 °C.
b. Un piston avec un filtre à pores de 40 micromètres est poussé par la pression du but du doigt dans l'ampoule de réaction en séparant ainsi le réactif adsorbant Protéine G Agarose sur le fond de l'ampoule de la solution aqueuse contenant le IgM à analyser, recueilli dans le réservoir du piston filtrant.
c. La solution filtrée recueillie dans le réservoir est aspirée par une pipette en plastique et transférée à une nouvelle ampoule attendant la détermination.

7. Une méthode selon la déclaration 1 qui emploie un réactif neutralisant qui élimine toute présence résiduelle d'anticorps interférents classe IgG, qui pourraient être restés dans la solution malgré le traitement avec protéine G Agarose. Ce passage est accompli par l'addition de 50 µl du réactif neutralisant directement dans les micro puits du module de réaction exactement avant l'addition de l'échantillon, dilué et prétraité comme décrit pour éviter des interférences dues aux anticorps classe IgG avant qu'ils peuvent réagir avec les peptides sur la phase solide.

8. Une méthode selon la déclaration 7 où le réactif neutralisant a la suivante formule :
| | |
|---|---|
| diNa- Phosphate dodecaidhrate | 0.01M |
| NaCI | 0.15M |
| Antisérum spécifique | 30% |
| HCl a Ph 7.4 +/-0.1 | |
